# EUROPEAN PATENT SPECIFICATION

(11) **EP 0 308 524 B1**
(45) Date of publication and mention of the grant of the patent: **26.04.1995**
(21) Application number: 87113812.9
(22) Date of filing: 22.09.1987
(51) Int. Cl.: A61M 15/00

(54) **Inhalation device and method**
Inhalationsvorrichtung und Methode
Dispositif d'inhalation et méthode

(43) Date of publication of application: 29.03.1989
(73) Proprietor: VORTRAN MEDICAL TECHNOLOGY, INC., Sacramento California 95815 (US)
(72) Inventor: Wong, Gordon A., M.D., Sacramento California 95831 (US); Lee, James I. C., Sacramento California 95825 (US); Peck, Gary Michael, Los Angeles California 90038 (US)
(74) Representative: Blumbach, Kramer & Partner

(56) References cited:
- EP-A- 0 132 352
- WO-A-86/01730
- FR-A- 474 904
- US-A- 3 848 807
- US-A- 4 241 877

## Description

The invention relates to an apparatus for dispersing a fluid according to the first part of claim 1. Such apparatus is used as applicator for medicans and, more specifically, as inhaler.

From FR-A-474,904 it is known to atomize thick liquids by forming films which are disrupted by pressure gas crossing the films. Feature b), b.4) may be identified as common with the invention.

For the treatment of conditions of the nose, throat or mouth, an aerosol applicator device is already known (EP-A-0,132,352) wherein an insert incorporating an atomizer nozzle as the outlet has a plurality of radial ribs or fins which leave spaces on its outer periphery which can be used as passages through which air may be inhaled by a patient using the device.

Devices for creating medicated aerosol using propellant and medication inside a canister are widely used. Although many medications for asthma are delivered by these devices, the wide range of particle size produced along with significant forward velocity results in discomfort and impaction in the patient's oropharynx. Many attempts have been made to decrease velocity and impaction by the use of "spacers". Spacers are straight tubes or bags that are attached to the aerosol device in an attempt to provide uniform particle size with reduced velocity and impaction. Collapsible bags have also been used. The current spacers are cumbersome and have not found wide-spread patient acceptance. However, clinical studies have shown spacers to be helpful in improving delivery of drugs to patients having difficulty coordinating the high velocity of the aerosol with inhalation. Increased retention of the aerosol medication has been a problem with these devices. Without spacers, the delivery of the aerosol is uncomfortable to patients because of the cooler temperature associated with the liquified propellant and medication.

US-A-4,241,877 shows a vortex generating device in FIGS. 5A, 5B and 6, i.e. a transducer having the features b), b.1) - b.6) and b.7).

The device shown in the drawings and described is suitable for relatively high mass flow rates, but would not be suitable for use with the propellant-charged canisters containing a relatively low propellant-to-medication liquid ratio. With such canisters, it is desirable to minimize the proportion of propellant in each dose and maximize the vortical action of the mixture while it flows through the transducer. Furthermore, it would be difficult and expensive to manufacture such a device and still provide the aerosol production capabilities required in conjunction with the propellant-charged canister.

A further known apparatus (WO 86/01730) shows the features a), b), b.1) - b.6), c), d), i.e. the first part of claim 1. It is contemplated that an entrainment valve may be placed behind the transducer with appropriate changes in the body of the inhaler to allow entrainment of air by the pressurized fluid. Adding air in such a manner will produce a non-uniform distribution of air and dispersed fluid.

The problem to be solved by invention is to create an apparatus for dispersing a fluid and for entraining same with a second fluid such that a symmetrical distribution of the first and second fluid within the total fluid flow is produced, and by a method for producing an aerosol.

This problem is solved by the combination of the features as recited in claims 1 or 8.

The inhalation device and method using vortex generating technology and combining it with entrainment on demand has resulted in a device capable of producing uniform particle size, comfortable temperature and without impaction. The reduced velocity will allow patients to easily coordinate actuations of the device with inhalation of the medicine. This will increase the amount of drug delivered to the lower respiratory tract and appropriate receptor sites instead of depositing in the oropharynx.

This arrangement provides an aerosol delivered at a very small or negligible velocity thereby minimizing impact of the aerosol on the patient. The device provides a mist having droplets which are uniform in size regardless of the variation in range of pressure provided through the liquified propellant in a propellant canister. This is also the case regardless of the amount medication atomized. The second chamber is approximately one-fourth the size of the spacer of the prior devices and is used to create the low velocity aerosol. The majority of droplets produced by the device are one-to-three microns which are important for delivery to the lower respiratory tract and receptor sites.

### BRIEF DESCRIPTION OF THE DRAWINGS

FIG. 1 is a schematic and sidesectional view of an apparatus for dispersing fluid in combination with a tube and sleeve forming an apparatus for applying medication as an aerosol;
FIG 2. is a schematic and partial side section of the apparatus of FIG. 1 showing the device with means for forming a passageway;
FIG. 3 is a schematic and side section of a transducer for the apparatus shown in FIG. 1 and showing flow passages through the transducer;
FIG. 4 is a schematic and side elevation view of the transducer of FIG. 3;
FIG. 5 is a schematic and sidesection of the device similar to that shown in FIG. 3 showing the pattern of fluid flow in the device;
FIG. 6 is a schematic and plan view of a resonant screen for use in the apparatus of FIG. 1 ; and
FIG. 7 is a schematic and partial side section view of an additional embodiment of an apparatus similar to that shown in FIG. 1.

### DETAILED DESCRIPTION

The description herein of the apparatus and its use includes a description of the method for applying medication as an aerosol. Reference is made to International Publication No. WO 85/02345, June 6, 1985, oriented to a Single Inlet Prepackaged Inhaler and to U.S. Patent No. 4,241,877, oriented to a Stable Vortex Generating Device. The material of these publications are incorporated herein.

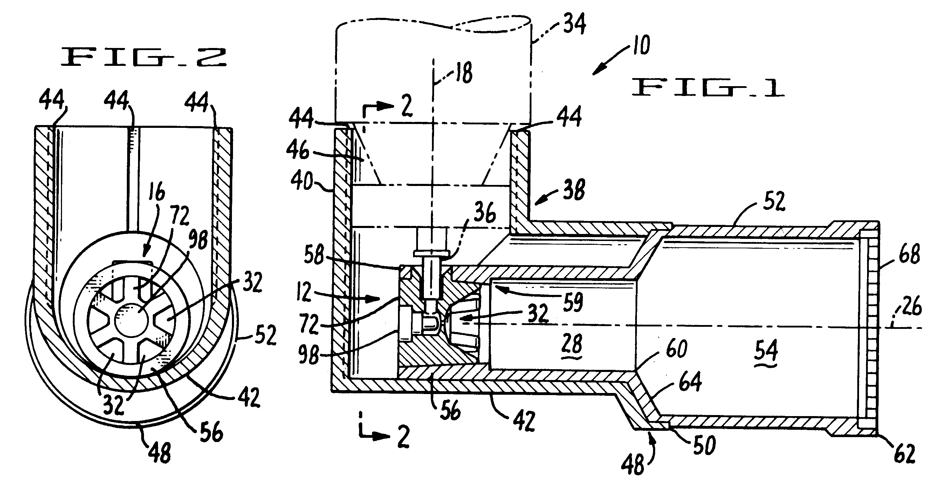

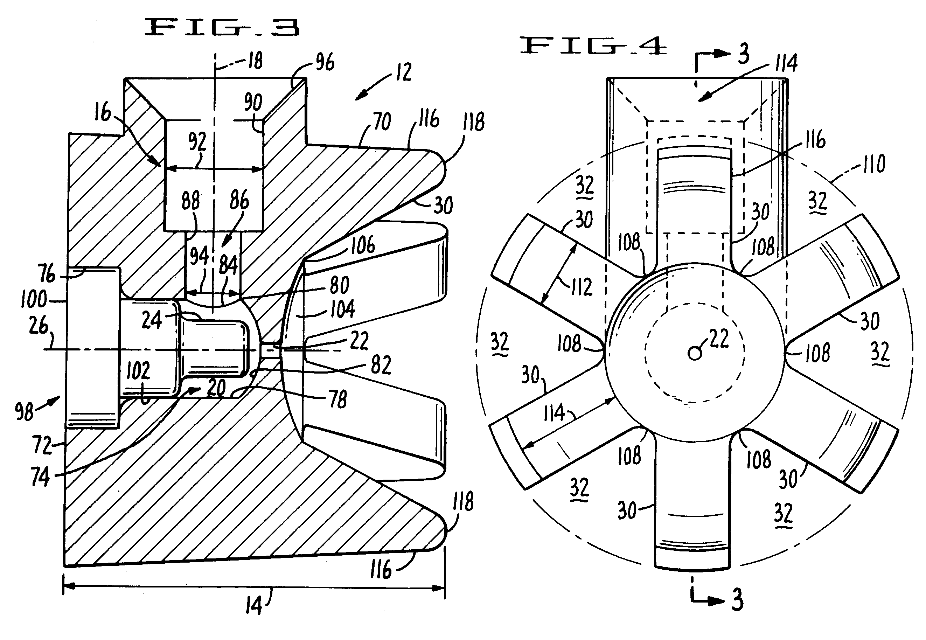

FIG. 1 of the present invention shows an apparatus in the form of an inhaler 10 for applying medication as an aerosol to a patient. A transducer 12, shown in detail in FIG. 3, includes a length 14 and has means in the form of an inlet 16 for supplying a first fluid (not shown). The inlet has a first axis 18. A first chamber 20 opens from the inlet 16 at a portion downstream from the inlet oriented vertically, as shown in FIG. 3. (Directional terms used herein such as vertical, horizontal and upward are intended to be used for reference to the drawings only and are not limitation on the structure or function of the device.) An outlet 22 is positioned downstream from the first chamber 20. A bluff body 24 is positioned between the inlet 16 and the outlet 22 and has an axis 26 contained in a plane (not shown) perpendicular to a plane (not shown) containing the first or inlet axis 18. A second chamber 28 in the form of an expansion chamber is positioned downstream from the outlet for expanding the fluid. Means in the form of spokes 30 for defining at least one passageway 32 is positioned between the second chamber 28 and the transducer 12 for passing a second fluid, such as air. The spokes 30 extend the length 14 of the transducer.

Consider now the apparatus in detail with respect to the drawings. The inhaler 10 is adapted to accept a container 34 of a mixture of propellant and medication (not shown). A nozzle 36 extends from one end of the container for dispensing the propellant and medication under pressure. The container has a structure and function similar to that of metered-dose containers generally known in the art.

The inhaler 10 includes a sleeve 38 for holding the container and for providing a support structure for the remainder of the apparatus. The sleeve has a vertical leg 40 and a horizontal leg 42 wherein the vertical leg is adapted to accept the nozzle end of the container 34. The vertical leg 40 is substantially hollow and cylindrical in crossection with a plurality of guide ridges 44 extending vertically and inwardly from the interior surface 46 of the vertical leg 40. The guide ridges support and retain the container 34 in a vertical position when placed in the inhaler 10.

In the embodiment of FIG. 1, the horizontal leg 42 of the sleeve 38 is tubular or substantially hollow cylindrical in transverse crossection. The inside diameter of the horizontal leg 42 has a diameter which is less than the inside diameter of the vertical leg 40 of the sleeve. The terminal end 48 of the sleeve includes a lip 50 extending outwardly from the lower portion of the horizontal leg 42. The upper portion of the horizontal leg extends essentially horizontally to the terminal end 48. The sleeve 38 is dimensioned to accept, in the horizontal leg 42, a tube 52, to be described more fully below, containing the transducer 12. The sleeve 38 also provides for the intake of air between the container 34 and the walls of the vertical leg 40 to allow entrainment of air through the passageways 32 about the transducer 12.

The tube 52 supports and retains the transducer 12 and includes the second expansion chamber 28 and a third expansion chamber 54 for expanding the aerosol product. The tube and transducer partially fit inside the horizontal leg 42 of the sleeve 38. The outside dimensions of the tube 52 are largely dictated by the outside dimensional requirements of the transducer, the internal dimensional requirements of the second and third expansion chambers, and by the coupling required between the terminal end 48 and the tube 52.

The tube 52 includes a transducer mount 56 for enclosing and supporting the circumferential surface of the transducer at an upstream end of the tube. The transducer mount includes a keyslot 58 for accepting the inlet of the transducer. The interior wall of the transducer mount converges in a downstream direction from 11.86 to 10.85 mm (0.467 to 0.427 inch) to assist in focusing the flow of the incoming air from the upstream end of the transducer passageways and to more easily hold the transducer. The sleeve and tube are preferably formed of a medical grade plastic, but may also be made from other materials.

The second expansion chamber 28 begins at the downstream terminus 59 of the transducer mount 56 and extends in a downstream direction to the beginning of the third expansion chamber 54. The inside diameter of the second expansion chamber preferably increases from 11.86 mm (0.467 inch) at the extreme upstream end of the second chamber 28 to approx. 12.2 mm (0.480 inch) at the extreme downstream end. The length of the expansion chamber 28 is preferably 18.9 mm (0.745 inch). The third expansion chamber 54 extends from the end 60 of the second expansion chamber to the downstream end 62 of the tube. The third expansion chamber begins with a diverging conical portion 64 having an inside surface extending outwardly and downstream at an angle of approximately 60° with respect to a horizontal axis 26. The inside surface of the remainder of the third expansion chamber is substantially cylindrical in crossection and preferably comprises an inside diameter of approximately 23.87 mm (0.940 inch). The length of the third expansion chamber is preferably 32.36 mm (1.274 inch) from the end 60 of the first expansion chamber to the downstream end 62 of the tube.

In the embodiment of FIG. 1, the tube includes means comprising apertures having lattice pieces in the form of screen 68 mounted to the downstream end 62 of the tube for setting up a standing wave pattern (not shown) in the expansion chambers, to assist in uniformly dispersing particles in the vortex and for serving as a drag brake to break up the remaining particles in the vortex. The screen is preferably formed of a nylon lattice having an extreme diameter of 24.89 mm (0.980 inch) and a square lattice opening dimension of 1.5 x 1.5 mm (0.060 x 0.060 inch). Each lattice member is preferably 0.3 mm (0.012 inch) across the face and 0.6 mm (0.024 inch) from one face of the screen to the other. In the preferred embodiment of the invention, the screen 68 is omitted.

The transducer will now be considered in more detail with respect to FIGS. 2-4. The transducer 12 has a transducer body 70. The transducer body includes a flat circular base 72 having formed therein a substantially cylindrical recess 74 and a counterbore 76.

The cylindrical recess 74 is formed by a cylindrical chamber wall 78 extending from the bottom of the counterbore 76 to a rim 80 in the chamber wall. The cylindrical recess is closed at the bottom thereof by a second chamber wall 82 constituting a portion of a sphere. The surface is curved to present minimum obstruction to flow once the vortices are created. The end of the bluff body 24 is curved for the same purpose.

An aperture 84 is formed in the cylindrical chamber wall 78 and is adjacent the rim 80 of the chamber wall. The aperture forms an opening for the inlet 16 to the first chamber 20. The inlet includes an inlet restriction 86 having a cylindrical wall 88 extending to the aperture 84.

The inlet further includes a counterbore 90 having a circular crossection and a diameter 92 greater than the diameter 94 of the aperture. The counterbore 90 accepts the nozzle 36 of the medication container 34. The inlet also includes a conically shaped surface 96 converging to the counterbore 90.

The first chamber 20 terminates at a downstream portion in the outlet 22. The outlet is substantially a pinhole having a circular transverse crossection.

A plug 98 is adapted to be placed in the cylindrical recess 74 and includes a round, flat head 100 to mate with the counterbore 76. The plug includes a reduced shaft 102 adjacent the head 100 for mating with the chamber wall 78. The reduced shaft serves to close off the cylindrical recess to form the first chamber 20. The bluff body 24 is a further reduced portion adjacent the reduced shaft extending into the first chamber 20 and past a plane defined by the rim 80 of the second chamber wall. Therefore, the end of the body 24 extends to a point between two parallel planes (not shown), the first defined by the rim 80 and the second defined by the upstream portion of outlet 22. The plug, including the head, reduced shaft and bluff body are oriented about the axis 26, which is perpendicular to axis 18. The bluff body extends across, in spaced apart relation with, the aperture 84.

The outlet 22 terminates in an expansion surface 104 defining a portion of a sphere and is curved so that the flow from the outlet is as free from disruption as possible. This helps to minimize deposition fluid on the transducer and maximize the proportion of medication delivered. The expansion surface extends downstream to a portion defining a rim 106.

The transducer also includes a plurality of spokes 30 defining a plurality of passageways 32. As shown in FIGS. 2 and 4, the spokes extend radially outward from an outer surface 108 of the transducer to an outer perimeter 110. The spokes 30 and therefore the transducer are supported by the transducer mount 56, shown in FIG. 1. In the preferred embodiment, there are six spokes oriented equidistant about the outer surface of the transducer. Each spoke has a width 112 from one side to the other, on opposite sides of a respective axis 114. Adjacent axes are preferably spaced approximately 60° apart. A top vertical spoke 116 is preferably oriented along a radius defined by the first axis 18, about which the inlet 16 is oriented. As shown in FIG. 3, the top vertical spoke extends in an upstream and a downstream direction from the inlet 16. The remaining spokes extend from the flat circular base 72 of the transducer substantially the length 14 of the transducer. Each spoke terminates in a preferably rounded end 118 at the downstream terminus of the transducer. Additionally, each spoke has a corresponding diametrically opposite spoke, forming a pair defining the perimeter of the transducer. The diameter of the perimeter of the transducer decreases in the downstream direction from the base 72 to the rounded ends 118 for engaging the transducer mount 56 in the tube 52.

The transducer and plug are preferably made of medical grade plastic. However, as with the sleeve and tube, it is possible that the transducer and plug be made from other materials. The dimensions of the transducer and plug are as follows: the diameter of the cylindrical recess 74 is 2.79 mm (0.110 inch); the diameter of the aperture 84 is 1.52 mm (0.060 inch); the diameter of the counterbore 90 is 2.74 mm (0.108 inch); the extreme diameter of the conically shaped surface 96 is 4.98 mm (0.196 inch); the length of the counterbore 90 conically shaped surface 96 is 4.32 mm (0.170 inch); the distance from the axis 26 to the extreme end of conically shaped surface 96 is 7.47 mm (0.294 inch); the height of the head 100 of plug 98 is 1.52 mm (0.060 inch); the combined length of the reduced shaft 102 and bluff body 24 is 3.56 mm (0.140 inch); the distance from the face 72 to the upstream portion of outlet 22 is 0.541 mm (0.0213 inch); the length of outlet 22 is 0.5 mm (0.020 inch); the inside diameter of outlet 22 ranges from 0.356 to 0.559 mm (0.014 to 0.022 inch); the angle with which the downstream spoke surface, defining a portion of a cone, mates with the horizontal is 30°; the length 14 is 10.54 mm (0.415 inch); the diameter of the expansion surface 104 is 5.08 mm (0.200 inch); the radius of the outer perimeter 110 is 5.94 mm (0.234 inch); the shortest radius of the perimeter of the transducer is 5.54 mm (0.218 inch); the width 112 is 2.03 mm (0.080 inch); the radius of curvature of the expansion surface 104 is 5.54 mm (0.218 inch).

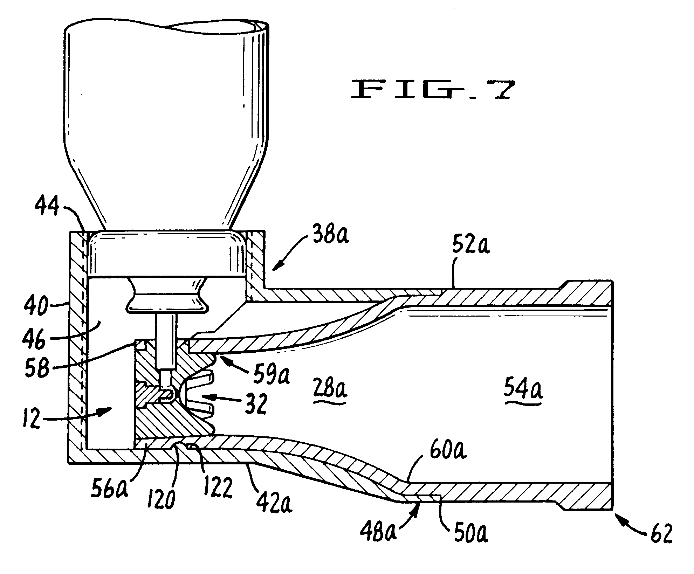

In a preferred embodiment of FIG. 7 the Inhaler 10 is similar to the Inhaler 10 of FIG. 1. Elements in FIG. 7 identical to those of FIG. 1 are labeled with the same reference numerals and have the same structure and function. The inhaler has a sleeve 38 having a horizontal leg 42a. The horizontal leg 42a of the sleeve is hollow and substantially circular in transverse crossection. The terminal end 48a of the horizontal leg includes a lip 50a extending outwardly from the lower portion of the horizontal leg. The lower portion extends substantially horizontally from the left side of the sleeve to a point midway along the sleeve and then downwardly in the right hand direction to the terminal end 48a. The upper portion of the sleeve extends substantially horizontally from the vertical leg to the terminal end 48a. With this configuration, the horizontal leg of the sleeve has a circular transverse crossection of increasing diameter from left to right beginning at the approximate midportion of the horizontal leg. The remainder of the surface of the horizontal leg is so formed to provide a smooth surface from the top portion of the horizontal leg to the bottom portion and from left to right. The terminal end 48a is also circular in crossection for accepting the tube 52a described more fully below.

The internal dimensions of the sleeve are configured to accept and hold the tube 52a. Specifically, a ridge 120 extends into the interior of the sleeve at a point to the left of the midportion for engaging a groove 122 in the tube 52a. The thickness of the wall of the horizontal leg is substantially constant between the left end and the midportion of the sleeve. Thereafter, the outside surface follows a relatively straight path outwardly and to the right while the inside surface follows a curved path whereby the thickness of the wall increases and then decreases toward the terminal end 48a. At any given point on the lower portion of the horizontal leg, the wall thickness is a maximum at the lower portion of the horizontal leg and decreases toward the upper portion of the horizontal leg.

The tube 52a supports and retains the transducer 12 and includes the second expansion chamber 28a and the third expansion chamber 54a for expanding and decreasing the forward velocity of the fluid. The tube and transducer partially fit inside the horizontal leg 42a of the sleeve 38a. The outside dimensions of the tube 52a are largely dictated by the outside dimensional requirements of the transducer, the internal dimensional requirements of the second and third expansion chambers and by the coupling required between the terminal end 48a and the tube 52a.

The tube includes a transducer mount 56a for enclosing and supporting the circumferential surface of the transducer at an upstream end of the tube. The transducer mount includes a keyslot 58 for accepting the inlet of the transducer. The transducer is substantially the same as described with respect to FIGS. 1-6. The interior wall of the transducer mount converges in a downstream direction to assist in focusing the flow of the incoming air from the upstream end of the transducer passageways to more easily hold the transducer.

The second expansion chamber 28a begins at the downstream terminus 59a of the transducer mount and extends in a downstream direction to the end 60a of the second expansion chamber and the beginning of the third expansion chamber. The walls of the second expansion chamber 28a preferably define a surface in the form of a portion of a hyperboloid of one sheet. As a result, the transverse crossectional shape of the second expansion chamber is substantially circular and increases in diameter from left to right. The inside diameter of the second expansion chamber preferably increases from approximately 11.86 mm (0.467 inch) at the extreme upstream end to approx. 23.88 mm (0.940 inch) at the extreme downstream end 60a. This provides a radius of curvature of approximately 50.8 mm (2 inch). The length of the second expansion chamber is preferably 25.27 mm (0.995 inch).

The third expansion chamber 54a extends from the end 60a of the second expansion chamber to the downstream end 62 of the tube. The third expansion chamber is substantially circular in transverse crossection having a substantially constant inside diameter of appr. 23.88 mm (0.940 inch) from beginning to end. The length of the third expansion chamber is preferably 27.63 mm (1.088 inch).

The transducer 12 is substantially the same as described above with respect to FIGS. 2-4.

The operation of the apparatus will now be described with respect to FIGS. 1-6. The inhaler 10 is assembled as would be apparent to one skilled in the art. A container 34 of a mixture of propellant, active medication and solvent is placed in an inverted fashion into the vertical leg of the sleeve 38. The outlet port of the nozzle 36 is then confluent with the inlet restriction 86 of the inlet 16. The staging chamber of the container 34 is filled with the appropriately metered dose of the mixture of propellant and medication. The container is then depressed relative to the counterbore 90 so that the nozzle is forced further into the mouth of the container. The mixture contained in the staging chamber is ejected under pressure of between 2-3.5 bar (30-50 psi), depending upon the particular container being used. The volume of the staging chamber may be approximately 0.05 cubic centimeters so that the total volume of the mixture of propellant and medication is approximately 0.05 cubic centimeters. As discussed in the '877 patent, the phenomenon of "cold boiling" and/or the resultant atomization of the liquid in the propellant/solvent mixture may occur when the injected mixture is pressurized and then released into the inlet restriction of the inlet 16. Preferably, the ratio of the gas pressure to the ambient pressure is greater than the critical pressure ratio. For example, the pressure of the mixture in the container may be approximately 3.5 bar (50 psi), gauge (psig). The propellant-to-liquid ratio may be typically 30:1 and the volume of output per dose may be 0.05 cubic centimeters.

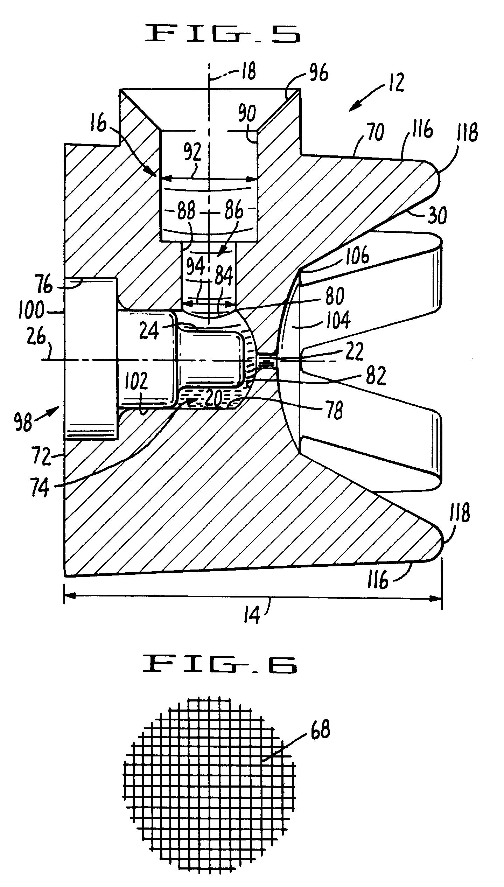

As a result of the high propellant and liquid pressure, the flow of gas is supersonic as it enters the inlet restriction 86. The ejected fluid expands into the inlet. The fluid flows along the inlet without further significant change in volume or velocity until reaching the aperture 84, where the mixture is then injected into the first chamber 20. The mixture again goes through expansion into the chamber 20. The presence of the bluff body in front of the supersonic fluid gives rise to a standing shockwave as shown in FIG. 5. A very large number of vortices is thereby generated about the bluff body. The potential energy, i.e., static pressure, of the gas supplied to the inlet, is in effect, partly converted to kinetic energy -- first as an increase in linear gas velocity, then as shock waves, and finally as vortices in the flow passage. The vortices are formed having axes generally parallel to the bluff body. It is estimated that the pressure at the bluff body is approximately 1.4-2.1 bar (20-30 psig).It is estimated that the tiny vortices are formed at a rate of approximately 40,000-50,000 vortices per second. This is due to the high velocity of the flow and to the dimensional compactness of the apparatus. Formation of a maximum number of vortices per unit/time is preferred. The number of vortices generated by the device is proportional to the gas velocity and the drag area across which the gas flows and the requisite coeffecient of drag. Because of the pressure difference between the inlet and the outlet 22, there is a pressure gradient toward the second chamber wall 82, along the outlet 22 and to the expansion surface 104. The individual vortices are focused along the second chamber wall toward the outlet 22. The spinning vortices are then compressed and forced by the pressure gradient into the outlet, followed by sudden expansion into a single large vortex (not shown).

The vortex activity from the bluff body to the final large vortex produces atomization of the liquid fraction in the liquid-propellant mixture so that medication contained in the liquid fraction is atomized into a mist substantially containing 1 to 3 micron-sized monodispersed droplets for entering the first expansion chamber 30. It was found that one massive vortex was formed by impinging the vortices on a rod and letting them escape through an annular chamber.

The single large exiting vortex is comprised of fluid flowing at supersonic velocity due to the compression of the vortices flowing through the orifice and due to the pressure difference across the orifice being greater than the critical pressure. The dose mixture went through a first supersonic flow regime, followed by expansion and creation of vortices. The vortices were then focused and forced out the outlet to the downstream side of the orifice where the pressure is approximately atmospheric. The exiting vortex represents a conversion of a substantial amount of static energy to dynamic energy. The vortex is spinning about the axis 26.

The single vortex includes a negative pressure at the center thereof due to the pressure differential across the orifice being greater or equal to the critical pressure ratio. It is found that there is enhanced atomization with this configuration.

The passageways allow entrainment of external air by the negative pressure zone and the single exiting vortex. Entrainment occurs along the passageways and mixing of the external air with the fluid from the outlet 22 occurs between the expansion surface 104 and a downstream portion of the inhaler. This has been found to decrease the rate of retention of the medication in the inhaler.

The second expansion chamber 28 provides for expansion of the vortex, thereby reducing the velocity and, therefore, mean free path of the individual medication particles and provides an area for confining the atomized vapor. The expansion and slowing of the particles in combination with the entrainment inhibits condensation of the individual particles and deposition of the particles along the chamber wall. The forward velocity of the particles is transferred to increased random motion of the individual particles. Additionally, it is believed that further atomization of the particles may occur to a limited extent.

As the vortex continues to the third expansion chamber 54, a like process occurs, without entrainment, whereby the forward velocity of the individual particles is further decreased and the vapor vortex is further expanded due to the increased crossectional area of the third expansion chamber. The effect of the increase in crossectional area on the vortex is the same as that from the expansion in the second expansion chamber. The third expansion chamber assists in storing the vapor in the vortex, slows the vortex in its forward movement and allows creation of greater vorticity when the fluid reaches the resonant screen, to be described more fully below.

The fluid in the vortex is then impacted on the resonant screen with several resulting effects. The screen resonates to set up a standing wave pattern in the expansion chambers to assist in uniformly dispersing particles in the vortex in the second expansion chamber. It also serves as a drag brake to break up the remaining particles in the vortex. This is enchanced by the fact that a force of drag on the fluid flow is more significant than the force due to the forward momentum of the fluid. Therefore, the effects due to the drag force predominate over the effect due to forward flow of the fluid. Additionally, as the vortex impacts the resonant screen, individual spinning vortices are produced in each square of the resonant screen due to the appearance to the fluid of individual bluff bodies oriented perpendicular to each other. There is also evidence of some vibration in the resonant screen. The position of the screen relative to the transducer and to the expansion chambers maximizes resonant effect and standing wave action, and also the production of the desired particle size, even after the fluid slows down in the expansion chambers. The final result, after impingement of the fluid on the resonant screen, is a soft, "warm", low-velocity mist or vapor to be inhaled by a patient. The mist will have minimal, if any, impact on the air passageways in the patient's mouth and lungs as the vapor is inhaled. The apparent temperature of the aerosol is more acceptable to the patient also.

The operation of the inhaler of FIG. 7 is substantially the same as that described with respect to FIGS. 1-6 except for the effect of the second expansion chamber 28 and the absence of the resonant screen. When the single exiting vortex and the entrained fluid enter the second expansion chamber, expansion of the mixture takes place gradually over the length of the second expansion chamber. Expansion takes place along the entire length of the second expansion chamber and it is believed that the configuration of the single existing vortex is maintained better than with the tube of FIG. 1 during the transition between the second and third expansion chambers. The resonant screen is omitted from the end of the tube to minimize deposition of medication of the lattice of the resonant screen.

The results obtained in the transducer combined with the expansion chambers are reproducable for any medication delivery canister presently marketed. The same output is still effected. When the inhaler is manufactured in a plastic molding process, the inhaler may be disposable after completion of a medication regimen from one or more medication canisters. The inhaler is easy to reliably manufacture and easily assembled with press-fit parts. The inhaler is compact and convenient to hold and carry. Entrainment of external air is provided to minimize medicine deposition in the expansion chamber and improve delivery to the patient.

It should be noted that the above are preferred configurations but others are foreseeable. The described embodiments of the invention are only considered to be preferred and illustrative of the inventive concept; the scope of the invention is not to be restricted to such embodiments. Various and numerous other arrangements may be devised by one skilled in the art without departing from the spirit and scope of the invention.

## Claims

1. An apparatus for producing an aerosol comprising
a) a device inlet (40) for receiving an external source (34) of a first fluid and propellant,
b) a transducer (12) for dispersing said first fluid in said propellant when it is expanding, including
b.1) a transducer inlet (16) fluidically connected to said external source (34),
b.2) a transducer chamber (20),
b.3) a bluff body (24) in the form of a rod extending in said transducer chamber (20) and defining a bluff axis (26), and
b.4) a transducer outlet (22);
b.5) said transducer inlet (16) having an inlet axis (18) and being connected to said transducer chamber (20) wherein said bluff body (24) is arranged with its bluff axis (26) substantially perpendicular to said inlet axis (18) so as to intersect same such that the first fluid impinges on said bluff body (24);
b.6) said transducer outlet (22) having an outlet axis (26) which is substantially parallel to said bluff axis (26) and being fluidically connected to said transducer chamber (20);
c) a device chamber (28) downstream of said transducer outlet (22),
d) a device outlet (62) for dispensing said first dispersed fluid,
characterized in that
b.7) said bluff body (24) terminates at an end which is located upstream of said transducer outlet (22)
and in that
e) an admitting means (30 32, 40, 44) is provided for admitting a second fluid into said device chamber (28) so as to mix said second fluid with said first fluid,
e.1) said device chamber (28) being a mixing chamber, e.2) said admitting means (30, 32, 40, 44) including passages (32) being arranged internal to said apparatus and having openings into said mixing chamber (28) which extend parallel to said outlet axis (26) for allowing the entrainment of said second fluid by said first fluid.

2. The apparatus according to claim 1
wherein said bluff body (24) forms the end of a plug (98) which forms an end wall of said transducer chamber (20).

3. The apparatus of claim 1 or 2
wherein said passages (32) are defined by a plurality of spaced apart spokes (30) being arranged and distributed around said transducer outlet (22) and being formed by wall portions of the body (70) of said transducer (12).

4. The apparatus according to claim 3
wherein at least two adjacent spokes (30) extend the entire length (14) of the transducer body (70).

5. The apparatus according to any of claims 1 through 4
wherein said external source is a container (34) filled with said first fluid and propellant and is received by said device inlet (40) such that a gap (44) is formed between container (34) and device inlet which is used as a port for said second fluid.

6. The apparatus according to any of claims 1 through 5
wherein an expansion surface (104) defining a portion of a sphere is arranged to connect said transducer outlet (22) with the openings of said passages (32) into said mixing chamber (28).

7. The apparatus according to any of claims 1 through 6 wherein an expansion chamber (54) is arranged between said mixing chamber (28) and said device outlet (62).

8. A method for producing an aerosol comprising the following steps:
a) providing a container (34) containing propellant and medication;
b) ejecting said propellant and medication from a nozzle (36) into a transducer chamber (20) via an inlet (16) and onto a bluff body (24) so as to create a great number of vortices of said propellant and medication;
c) ejecting said propellant and medication from said transducer chamber (20) via an outlet (22) in a direction (26) substantially perpendicular to the direction (18) of ejection in step b) into a mixing chamber (28) thereby atomizing the liquid fraction in the medication-propellant mixture and producing an expanding stream of aerosol;
d) flowing said expanding stream of aerosol across a plurality of of passage ways (32) so as to produce vacuum and suck air through said passageways;
e) entraining and mixing said aerosol with said air in said mixing chamber (28) to produce an entrained aerosol for inhalation of said medication.

9. The method of claim 8
wherein said vortices are focused along a spherical wall (82) of said transducer chamber (20) toward said transducer outlet (22) and said propellant and medication is suddenly expanded to form a single large vortex flow in said mixing chamber (28) around the axis of ejection (26).

10. The method of claim 8 or 9
wherein said container (34) provides for a pressure between 2 and 3,5 bars.

11. The method of claim 10
wherein the flow in step b) is supersonic.

12. The method of claim 11
wherein a standing shockwave is produced in said transducer chamber (20).

## Patentansprüche

1. Gerät zur Erzeugung eines Aerosols
a) mit einem Vorrichtungseinlaß (40) zur Aufnahme einer externen Quelle (34) für ein erstes Fluid und Treibmittel,
b) mit einem Wandler (12) zum Dispergieren des ersten Fluids in dem Treibmittel, wenn dieses sich ausdehnt, wobei der Wandler umfaßt:
b1) einen Wandlereinlaß (16), der strömungsmäßig an die externe Quelle (34) angeschlossen ist,
b2) eine Wandlerkammer (20),
b3) einen Prallkörper (24) in Form eines Stabes, der sich in die Wandlerkammer (20) erstreckt und eine Aufprallachse (26) definiert,
b4) einen Wandlerauslaß (22),
b5) der Wandlereinlaß (16) besitzt eine Einlaßachse (18) und ist an die Wandlerkammer (20) angeschlossen, worin der Prallkörper (24) mit seiner Aufprallachse (26) im wesentlichen senkrecht zu der Einlaßachse (18) angeordnet ist, so daß dieser dieselbe schneidet, derart, daß das erste Fluid auf den Prallkörper (24) auftrifft,
b6) der Wandlerauslaß (22) besitzt eine Auslaßachse (26), die im wesentlichen parallel zu der Aufprallachse (26) ist und strömungsmäßig an der Wandlerkammer (20) angeschlossen ist,
c) mit einer Vorrichtungskammer (28) stromab vom Wandlerauslaß (22),
d) mit einem Vorrichtungsauslaß (62) zur Abgabe des dispergierten Fluids,
dadurch gekennzeichnet,
b7) daß der Prallkörper (24) an einem Ende endigt, das stromauf zu dem Wandlerauslaß (22) angeordnet ist, und
e) daß eine Zugangseinrichtung (30,32,40,44) zur Zufuhr eines zweiten Fluids in die Vorrichtungskammer (28) vorgesehen ist, um das zweite Fluid mit dem ersten Fluid zu mischen, wobei
e1) die Vorrichtungskammer (28) ein Mischungsraum ist,
e2) die Zugangseinrichtung (30,32,40,44) Durchtrittskanäle (32) umfaßt, die innerhalb des Gerätes angeordnet sind und Öffnungen in den Mischungsraum (28) hinein besitzen, die sich parallel zu der Auslaßachse (26) erstrecken, um das Mitreißen des zweiten Fluids durch das erste Fluid zu ermöglichen.

2. Gerät gemäß Anspruch 1,
dadurch gekennzeichnet, daß der Prallkörper (24) das Ende eines Stopfens (98) bildet, der eine Abschlußwand der Wandlerkammer (20) bildet.

3. Vorrichtung gemäß Anspruch 1 oder 2,
dadurch gekennzeichnet, daß die Durchtrittskanäle (32) durch eine Vielzahl von im Abstand angeordneten Speichen (30), die um den Wandlerauslaß (22) angeordnet und verteilt sind sowie durch Gehäusewandteile (70) des Wandlers (12) begrenzt werden.

4. Vorrichtung gemäß Anspruch 3,
dadurch gekennzeichnet, daß wenigstens zwei benachbarte Speichen (30) sich über die gesamte Länge (14) des Wandlergehäuses (70) erstrecken.

5. Vorrichtung gemäß einem der vorhergehenden Ansprüche 1 bis 4,
dadurch gekennzeichnet, daß die externe Quelle ein Behälter (34) ist, der mit dem ersten Fluid und einem Treibmittel gefüllt ist, und der von dem Vorrichtungseinlaß (40) aufgenommen wird, derart, daß ein Spalt (44) zwischen dem Behälter (34) und dem Vorrichtungseinlaß gebildet wird, der als Einlaß für das zweite Fluid benutzt wird.

6. Vorrichtung gemäß einem der Ansprüche 1 bis 5,
dadurch gekennzeichnet, daß eine Expansionsoberfläche (104), die einen Teil einer Kugel definiert, zur Verbindung des Wandlerauslasses (22) mit den in den Mischungsraum (28) führenden Durchtrittskanal (32) angeordnet ist.

7. Vorrichtung gemäß einem der Ansprüche 1 bis 6,
dadurch gekennzeichnet, daß ein Expansionsraum (54) zwischen dem Mischungsraum (28) und dem Vorrichtungsauslaß (62) angeordnet ist.

8. Verfahren zur Erzeugung eines Aerosols, das die folgenden Schritte umfaßt:
a) Bereitstellen eines Behälters (34), der Treibmittel und Arzneimittel enthält;
b) Ausstoßen des Treibmittels und des Arzneimittels aus einer Düse (36) in eine Wandlerkammer (20) über einen Einlaß (16) und auf einen Prallkörper (24), derart, daß eine große Anzahl von Wirbeln des Treibmittels und des Arzneimittels erzeugt werden;
c) Ausstoßen des Treibmittels und des Arzneimittels aus der Wandlerkammer (20) über einen Auslaß (22) in einer Richtung (26), die im wesentlichen senkrecht zu der Ausstoßrichtung (18) in Schritt b) ist, in einen Mischungsraum (28), wobei die flüssige Fraktion der Arznei-/Treibmittelmischung zerstäubt und ein sich ausbreitender Aerosolstrom erzeugt wird;
d) Strömenlassen des sich ausbreitenden Aerosolstroms parallel zu einer Vielzahl von Durchtrittskanälen (32), so daß dort ein Vakuum erzeugt wird und Luft durch die Durchtrittskanäle gezogen wird;
e) Mitreißen und Mischen des Aerosols mit der Luft in dem Mischungsraum (28), um ein mitgeführtes Aerosol zur Inhalation des Arzneimittels zu erzeugen.

9. Verfahren nach Anspruch 8,
dadurch gekennzeichnet, daß die Wirbel entlang einer sphärischen Wand (82) des Wandlerraums (20) zum Wandlerauslaß (22) hin fokussiert werden und das Treib- und Arzneimittel plötzlich expandiert, um eine einzige große Wirbelströmung in den Mischungsraum (28) um die Ausstoßachse (26) zu bilden.

10. Verfahren nach Anspruch 8 oder 9,
dadurch gekennzeichnet, daß der Behälter (34) einen Druck zwischen 2 und 3,5 bar bereitstellt.

11. Verfahren nach Anspruch 10,
dadurch gekennzeichnet, daß die Strömung in Schritt b) eine Überschallströmung ist.

12. Verfahren nach Anspruch 11,
dadurch gekennzeichnet, daß eine stehende Schockwelle in dem Wandlerraum (20) erzeugt wird.

## Revendications

1. Dispositif de production d'un aérosol comprenant :
a) une entrée de dispositif (40) pour recevoir une source externe (34) d'un premier fluide et d'un propulseur;
b) un transducteur (12) pour disperser ledit premier fluide dans ledit propulseur lors de sa dilatation, comprenant :
- une entrée de transducteur (16) raccordée hydrauliquement à ladite source externe (34);
- une chambre de transducteur (20);
- un corps non profilé (24) sous la forme d'une tige s'étendant dans ladite chambre de transducteur (20) et définissant un axe non profilé (26);
- une sortie de transducteur (22);
- ladite entrée de transducteur (16) possédant un axe d'entrée (18) et étant raccordée à ladite chambre de transducteur (20) dans laquelle ledit corps non profilé (24) est placé, son axe non profilé (26) étant pratiquement perpendiculaire audit axe d'entrée (18) de façon à le couper de telle façon que le premier fluide frappe ledit corps non profilé (24); et
- ladite sortie de transducteur (22) possédant un axe de sortie (26) pratiquement parallèle audit axe non profilé (26) et étant raccordé hydrauliquement à ladite chambre de transducteur (20);
c) une chambre de dispositif (28) en aval de ladite sortie de transducteur (22); et
d) une sortie de dispositif (62) pour la distribution dudit premier fluide dispersé,
dispositif caractérisé en ce que :
- ledit corps non profilé (24) se termine à une extrémité située en amont de ladite sortie de transducteur (22);
et en ce que :
e) un moyen d'admission (30, 32, 40, 44) est prévu pour admettre un second fluide dans ladite chambre de dispositif (28) de façon à mélanger ledit second fluide audit premier fluide:
- ladite chambre de dispositif (28) étant une chambre de mélange;
- ledit moyen d'admission (30, 32, 40, 44) comprenant des passages (32) internes audit dispositif et possédant des ouvertures dans ladite chambre de mélange (28) s'étendant parallèles audit axe de sortie (26) pour permettre l'entraînement dudit second fluide par ledit premier fluide.

2. Dispositif selon la revendication 1, dans lequel ledit corps non profilé (24) forme l'extrémité d'un bouchon (98) formant une paroi d'extrémité de ladite chambre de transducteur (20).

3. Dispositif selon la revendication 1 ou 2, dans lequel lesdits passages (32) sont définis par une pluralité de rayons espacés (30) disposés et répartis autour de ladite sortie de transducteur (22) et formés par des parties de paroi du corps (70) dudit transducteur (12).

4. Dispositif selon la revendication 3, dans lequel au moins deux rayons adjacents (30) s'étendent sur toute la longueur (14) du corps de transducteur (70).

5. Dispositif selon l'une quelconque des revendications 1 à 4, dans lequel ladite source externe est un récipient (34) rempli desdits premier fluide et propulseur et est reçue par ladite entrée de dispositif (40) de telle façon qu'un intervalle (44) soit formé entre le récipient (34) et l'entrée de dispositif qui est utilisée comme orifice pour ledit second fluide.

6. Dispositif selon l'une quelconque des revendications 1 à 5, dans lequel une surface de dilatation (104) définissant une partie d'une sphère est disposée pour raccordée ladite sortie de transducteur (22) aux ouvertures desdits passages (32) dans ladite chambre de mélange (28).

7. Dispositif selon l'une quelconque des revendications 1 à 6, dans lequel une chambre d'expansion (54) est placée entre ladite chambre de mélange (28) et ladite sortie de dispositif (62).

8. Procédé de production d'un aérosol comprenant les étapes suivantes :
a) la prévision d'un récipient ((34) contenant un propulseur et un médicament;
b) l'éjection desdits propulseur et médicament par une buse (36) dans une chambre de transducteur (20), via une entrée (16), et sur un corps non profilé (24) de façon à créer un grand nombre de vortex desdits propulseur et médicament;
c) l'éjection desdits propulseur et médicament de ladite chambre de transducteur (20), via une sortie (22), dans une direction (26) pratiquement perpendiculaire à la direction (18) d'éjection à l'étape b) dans une chambre de mélange (28), pulvérisant ainsi la fraction liquide du mélange médicament/propulseur et produisant un courant dilaté d'aérosol;
d) l'écoulement dudit courant dilaté d'aérosol par une pluralité de passages (32) de façon à produire un vide et de l'air d'aspiration par lesdits passages; et
e) l'entraînement et le mélange dudit aérosol avec ledit air dans ladite chambre de mélange (28) pour produire un aérosol entraîné pour l'inhalation dudit médicament.

9. Procédé selon la revendication 8, selon lequel lesdits vortex sont focalisés le long d'une paroi sphérique (82) de ladite chambre de transducteur (20) vers ladite sortie de transducteur (22) et lesdits propulseur et médicament se dilatent brusquement pour former un seul grand écoulement en vortex dans ladite chambre de mélange (28) autour de l'axe d'éjection (26).

10. Procédé selon la revendication 8 ou 9, selon lequel ledit récipient (34) est prévu pour une pression comprise entre 2 et 3,5 bars.

11. Procédé selon la revendication 10, selon lequel l'écoulement à l'étape b) est supersonique.

12. Procédé selon la revendication 11, selon lequel une onde de choc droite est produite dans ladite chambre de transducteur (20).
